# EUROPEAN PATENT SPECIFICATION

(11) **EP 3 954 409 B1**
(45) Date of publication and mention of the grant of the patent: **13.08.2025**
(21) Application number: 21188476.2
(22) Date of filing: 29.07.2021
(51) Int. Cl.: A61M 5/00, A61M 5/142

(54) **PACKAGING CONTAINER**
VERPACKUNGSBEHÄLTER
CONTENEUR D'EMBALLAGE

(30) Priority: 29.07.2020 JP 2020128265
(43) Date of publication of application: 16.02.2022
(73) Proprietor: TERUMO Kabushiki Kaisha, Tokyo 151-0072 (JP)
(72) Inventor: HASUMI, Kiyoaki, Showa-cho, 409-3853 (JP)
(74) Representative: Casalonga

(56) References cited:
- WO-A1-2018/227066
- JP-A- 2001 104 475
- US-A1- 2018 280 609

## Description

### BACKGROUND

### Technical Field

The present invention relates to a packaging container in which a stored object is stored in a recess formed in a resin sheet and the recess is sealed with a sealing film.
In particular, the present invention relates to a packaging container according to the preamble of independent claim 1, such as it is e.g. known from WO2018/227066 A1 or JP 2001 104475 A.

There has been conventionally proposed a packaging container in which a medical device is stored in a recess formed on a resin sheet and the recess is sealed with a flat sealing film such as a resin film (JP 2011-005182 A). Such a packaging container is also called a blister package or a blister case, and is used for packaging of, for example, a prefilled syringe, a drug solution administration device used by attaching to a body, and the like.

### SUMMARY

In some medical devices, the function is impaired if the medical device is touched when taken out from the packaging container. However, in the current packaging container, the entire area around the recess of the sealing film can be opened with a uniform force, and the user can peel off the sealing film all at once. In this case, all the accommodated medical devices are exposed at a time, and the user can touch all the parts of the medical devices. Therefore, there is a risk that the user grasps the wrong part, which may deteriorate the function of the medical device.

In addition, the recent spread of home treatment has increased an opportunity for patients themselves to operate medical devices. There is a concern about an increase in erroneous handling when users unfamiliar with handling of the medical devices take out the medical devices from packaging containers. Also in some products other than medical devices, the function may deteriorate if an inappropriate part is touched when taken out from packaging containers.

Therefore, there is a demand for a packaging container in which the user can take out the stored object without touching a wrong part.

**In order to solve this problem, the invention provides a packaging container according to independent claim 1. The dependent claims relate to advantageous embodiments.**

According to the packaging container of the **invention** the protection target portion is protected by providing the part surrounding the protection target portion of the stored object with a difficult-to-peel portion, which is difficult to peel off the sealing film, and hence it is possible to take out the stored object without the user touching a wrong portion.

### BRIEF DESCRIPTION OF DRAWINGS

FIG. 1 is a front view of a packaging container according to a first embodiment;
FIG. 2 is an exploded perspective view of the packaging container of FIG. 1;
FIG. 3 is an explanatory view of a housing attachment material and a protection sheet of the packaging container of FIG. 2;
FIG. 4 is an explanatory view of a seal portion in which a sealing film is bonded to the container body of FIG. 1; and
FIG. 5A is a cross-sectional view of a packaging container according to a second embodiment, and FIG. 5B is a plan view showing a shape of a seal portion of the packaging container of FIG. 5A.

### DETAILED DESCRIPTION

Preferred embodiments of the present invention will be described in detail below with reference to the accompanying drawings. Note that, for convenience of description, some dimension ratios in the drawings are exaggerated and different from actual ratios.

### (First Embodiment)

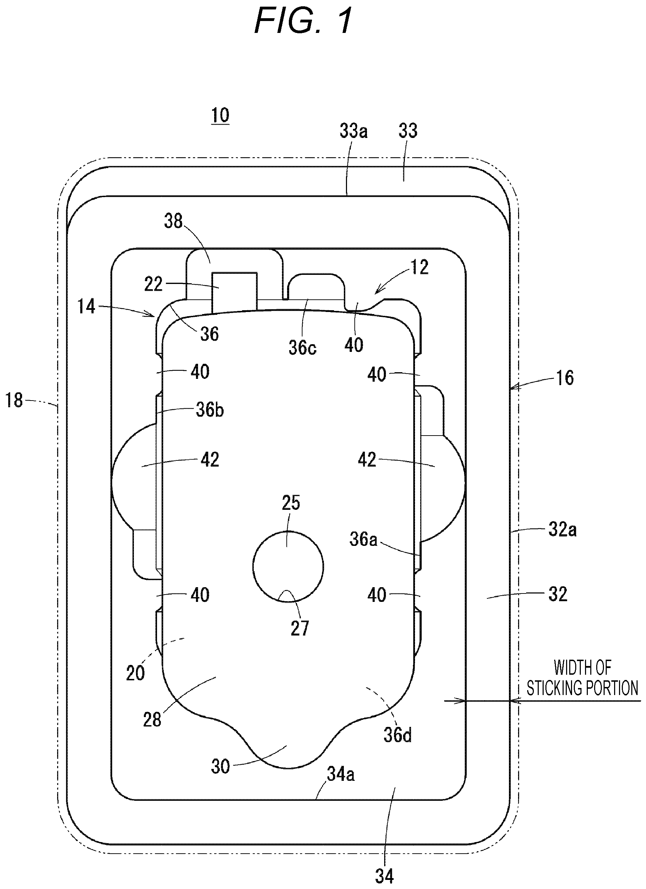

The packaging container 10 according to the present embodiment illustrated in FIG. 1 is used to accommodate a small drug solution administration device 12 (medical device) of a patch type to be attached to the body of a patient. The packaging container 10 has a container body 16 in which a recess 14 capable of accommodating the drug solution administration device 12 is formed on a transparent thick resin sheet, and a sealing film 18 stuck to the container body 16 and sealing the drug solution administration device 12 accommodated in the recess 14. In the present embodiment, the drug solution administration device 12, which is a type of medical devices, is described as a stored object accommodated in the packaging container 10. However, the present invention is not limited thereto. The packaging container 10 may target various types of devices having portions that may deteriorate in function when touched such as various types of sensors and fragile structures (needle tubes).

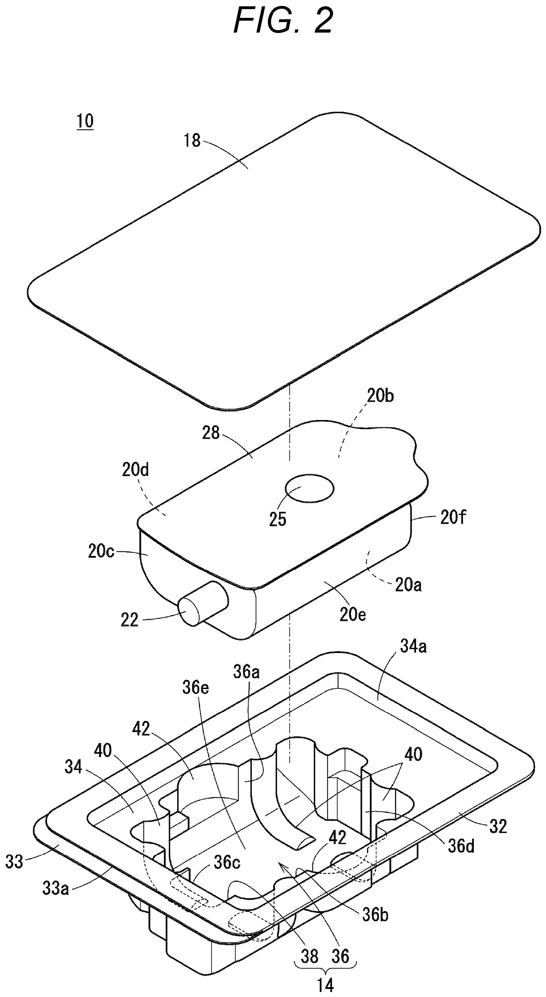

As shown in FIG. 2, the drug solution administration device 12 has a box-shaped housing 20. The inside of the housing 20 is provided with a cylindrical body accommodating a drug solution, a pusher that sends out the drug solution, and a drive mechanism that drives the pusher. The drug solution administration device 12 is used for continuously or intermittently administering a drug solution into a living body for a relatively long time (e.g., about several minutes to several hours).

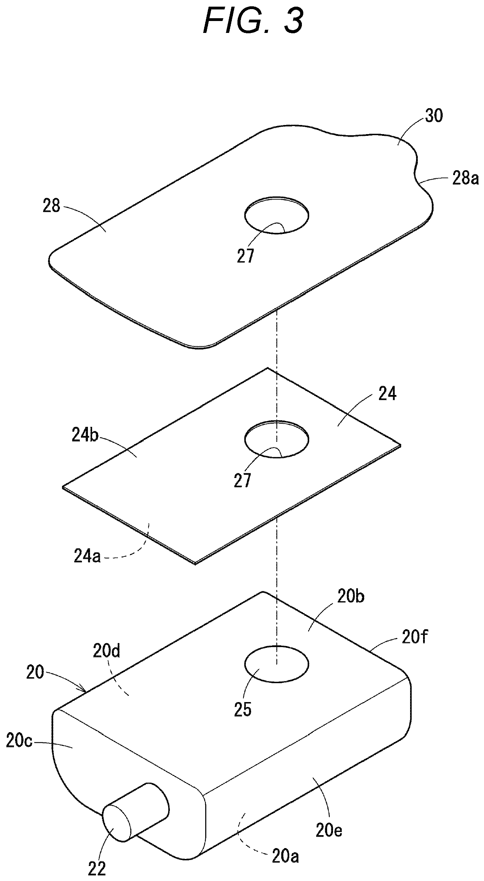

As shown in FIG. 3, the housing 20 has a rectangular front surface 20a and a rear surface 20b, and four side surfaces 20c to 20f. The front surface 20a and the rear surface 20b are formed to have an area larger than the side surfaces 20c and 20d. The side surface 20c is protrusively provided with a connection port 22 for connecting a patch type needle tube or the like. The rear surface 20b of the housing 20 is provided with a power switch 25. The power switch 25 is a switch for starting the administration operation of the drug solution administration device 12, and is disposed facing the body of the patient after the power switch 25 is depressed.

A housing attachment material 24 is configured such that one joining surface 24a is joined to the housing 20 and the other adhesion surface 24b can be stuck to the skin. The housing attachment material 24 is formed to have a shape identical to or larger than the rear surface 20b of the drug solution administration device 12 in order to secure the peeling strength with the skin.

A protection sheet 28 is attached to the housing attachment material 24 in order to protect the adhesion surface 24b until immediately before the use and to prevent the adhesiveness of the adhesion surface 24b from deteriorating. The protection sheet 28 is formed in a shape covering the entire area of the adhesion surface 24b. The protection sheet 28 is removed from the housing attachment material 24 immediately before the use of the drug solution administration device 12, and one end 28a (end on the side surface 20f side) of the protection sheet 28 is provided with a peeling start portion 30 separated from the housing attachment material 24.

The peeling start portion 30 is formed integrally with the protection sheet 28, and is formed as a portion where the protection sheet 28 bulges outward relative to the housing attachment material 24. The peeling start portion 30 is separated from the adhesion surface 24b of the housing attachment material 24, and is not in contact with the adhesion surface 24b. The user can easily peel the protection sheet 28 from the housing attachment material 24 by pinching the peeling start portion 30 and pulling the protection sheet 28 in the direction of peeling off the protection sheet 28 from the housing attachment material 24.

The rear surface 20b of the housing 20 is provided with the power switch 25 for starting drive of the drug solution administration device 12. The housing attachment material 24 and the protection sheet 28 in a part corresponding to the power switch 25 is provided with a circular notch hole 27 for enabling visual recognition and operation of the power switch 25.

On the other hand, as shown in FIG. 1, the container body 16 is provided with the recess 14, a planar sticking portion 32 surrounding the periphery of the recess 14, and an intermediate portion 34 formed between the recess 14 and the sticking portion 32. The recess 14 has a body accommodation portion 36 that accommodates the housing 20 of the drug solution administration device 12 and a port accommodation portion 38 that accommodates the connection port 22.

The body accommodation portion 36 is formed in a rectangular shape surrounded by four side surfaces 36a to 36d and a bottom surface 36e. The plane dimension of the body accommodation portion 36 is formed larger than the plane dimensions of the front surface 20a and the rear surface 20b of the housing 20. The depth of the body accommodation portion 36 (the depth from the intermediate portion 34 to the bottom surface 36e) is formed larger than the thickness of the housing 20 (the distance between the front surface 20a and the rear surface 20b), and the entire housing 20 is accommodated in the body accommodation portion 36.

The body accommodation portion 36 is provided with a plurality of protrusions 40 protruding from the side surfaces 36a and 36b and the bottom surface 36e toward the housing 20. The housing 20 is retained in the body accommodation portion 36 by being held between four protrusions 40 protruding from the side surfaces 36a and 36b. The housing 20 is retained in a state of coming off from the side surfaces 36a and 36b and the bottom surface 36e by abutting on the protrusion 40.

On the side surfaces 36a and 36b of the body accommodation portion 36, grasping recesses 42 are formed so as to make it easy to pinch the housing 20 of the drug solution administration device 12 when the housing 20 is taken out of the container body 16. The grasping recess 42 forms a gap into which the user can insert his fingertip. In a part other than the grasping recess 42, the gap between the body accommodation portion 36 and the drug solution administration device 12 is formed to have a width in which the user's finger cannot be inserted.

The port accommodation portion 38 is formed in a part of the recess 14 where the side surface 20c of the housing 20 is disposed. The port accommodation portion 38 is formed in a shape larger than the connection port 22, and the connection port 22 is accommodated in the port accommodation portion 38 in a state of being separated from the surface of the port accommodation portion 38. In order to prevent the user from touching the connection port 22 and damaging the connection port 22 when taking out the drug solution administration device 12, it is preferable that the gap between the port accommodation portion 38 and the connection port 22 has a width that does not allow the user's fingertip to enter.

The intermediate portion 34 is a part formed so as to surround the periphery of the recess 14, and has a plane parallel to the sticking portion 32. The inner peripheral edge of the intermediate portion 34 is connected to the recess 14. The outer peripheral edge of the intermediate portion 34 is connected to the sticking portion 32 via a step portion 34a. The intermediate portion 34 is formed lower than the sticking portion 32 via the step portion 34a. The intermediate portion 34 forms a space that accommodates the housing attachment material 24 and the protection sheet 28 of the drug solution administration device 12 between the intermediate portion 34 and the sealing film 18.

The sticking portion 32 is formed in a rectangular shape along the outer periphery of the container body 16. The distance between the step portion 34a and an outer peripheral edge 32a, which is the width of the sticking portion 32, is substantially constant throughout the entire circumference. A separation portion 33 is formed at an end of the sticking portion 32 on the port accommodation portion 38 side. The separation portion 33 is connected to the sticking portion 32 via the step portion 33a, and is formed at a position lower than the sticking portion 32. The separation portion 33 is separated from the sealing film 18 when the sealing film 18 described later is bonded to the sticking portion 32, and becomes a portion easily graspable of the sealing film 18 when the user starts peeling the sealing film 18.

In the container body 16, the recess 14, the sticking portion 32, and the intermediate portion 34 are integrally formed by the resin sheet. As the resin sheet, for example, a transparent sheet body made of PET resin can be used. In this case, the thickness of the resin sheet can be, for example, as thick as about 0.8 mm.

In the recess 14 of the container body 16, the drug solution administration device 12 is accommodated with the front surface 20a of the housing 20 facing the bottom surface 36e of the recess 14. The housing attachment material 24 and the protection sheet 28 of the drug solution administration device 12 are spread and accommodated in the intermediate portion 34 of the container body 16. At this time, as shown in FIG. 1, the peeling start portion 30 of the protection sheet 28 is disposed so as to extend to the intermediate portion 34 of the body accommodation portion 36 on the side surface 36d side.

The peeling start portion 30 of the protection sheet 28 is a portion easily peeled off when the drug solution administration device 12 is taken out from the packaging container 10, and is a protection target portion that requires protection when the drug solution administration device 12 is opened. If the protection sheet 28 is peeled off at the time of opening, the adhesion surface 24b is not protected and the adhesive power is lowered, and there is a risk that the drug solution administration device 12 falls off from the patient's skin during use. The peeling start portion 30 is a portion that is likely to be pulled by the user when taking out the drug solution administration device 12 from the packaging container 10. The protection sheet 28 is bonded to the housing attachment material 24 with a relatively weak bonding power. Hence, when the user pulls the peeling start portion 30, the protection sheet 28 is peeled off before the drug solution administration device 12 is taken out of the packaging container 10. Therefore, the peeling start portion 30 is a portion that needs protection when the drug solution administration device 12 is taken out of the packaging container 10.

As shown in FIG. 1, the sealing film 18 is formed in a rectangular shape having the dimensions same as the container body 16. The sealing film 18 is made of, for example, a thin transparent film made of polyethylene resin or the like. The sealing film 18 is peelably bonded to the sticking portion 32 of the container body 16.

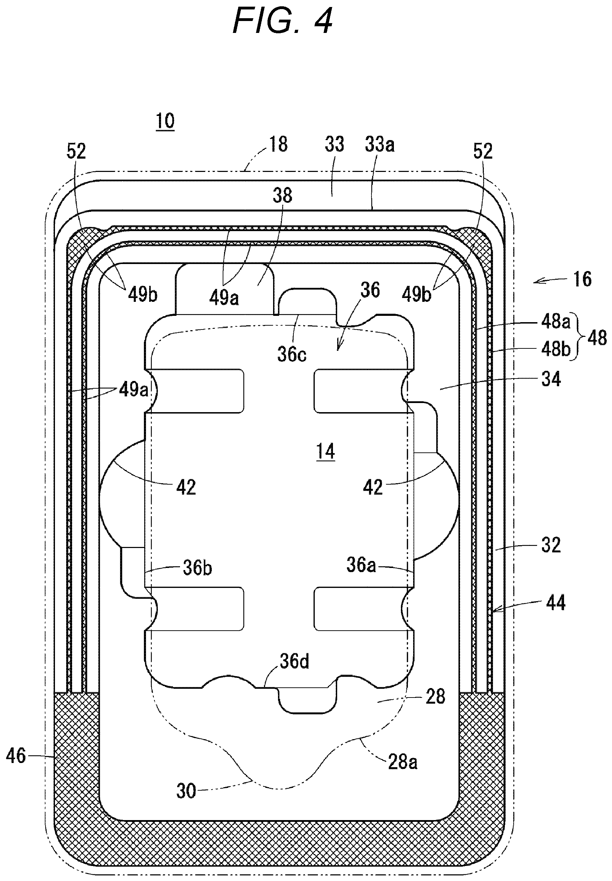

As shown in FIG. 4, the sealing film 18 extends along the sticking portion 32 and is bonded to the sticking portion 32 at a seal portion 44 formed so as to surround the entire periphery of the recess 14. The seal portion 44 can be formed by sandwiching the sealing film 18 and the sticking portion 32 with a mold of a predetermined shape, locally heating, and fusing in a state where the sealing film 18 is superposed on the sticking portion 32.

The seal portion 44 has a constant bonding power per unit area. However, the seal portion 44 of the present embodiment is provided with a difficult-to-peel portion 46 formed wide throughout the entire area of the width of the sticking portion 32 (the distance between the step portion 34a and the outer peripheral edge 32a), and an easy-to-peel portion 48 formed in linear patterns 48a and 48b having a width narrower than the width of the sticking portion 32.

Since the difficult-to-peel portion 46 is bonded to the sticking portion 32 with a wide bonding area, the peelable portion is bonded by a bonding power higher than that of the easy-to-peel portion 48, and the difficult-to-peel portion 46 is more difficult to peel compared with the easy-to-peel portion 48. The difficult-to-peel portion 46 is formed in a C-shape along the sticking portion 32 of the part surrounding the peeling start portion 30 of the protection sheet 28 of the drug solution administration device 12.

The easy-to-peel portion 48 is bonded to the sticking portion 32 with a width narrower than the width of the sticking portion 32, has a smaller bonding area than that of the difficult-to-peel portion 46, and is bonded by a relatively weak bonding power. Therefore, the easy-to-peel portion 48 can be peeled off more easily than the difficult-to-peel portion 46. The easy-to-peel portion 48 includes the two parallel linear patterns 48a and 48b. The linear patterns 48a and 48b include a straight part 49a and a bent portion 49b connecting adjacent straight parts 49a.

Of the two linear patterns 48a and 48b, the linear pattern 48a is a pattern formed inside and the linear pattern 48b is a pattern formed outside. The two linear patterns 48a and 48b are disposed so as to be separated from each other with a gap of a fixed width so as not to affect the other even if one is peeled off. The width of each of the linear patterns 48a and 48b can be 0.1 to 1 mm, for example.

The bent portion 49b of the outer linear pattern 48b is formed with a bulging portion 52 curved and bulging outward in an arc shape. The bulging portion 52 is formed as a bonding part similar to the linear pattern 48b, but is a portion where tensile force acting on the sealing film 18 is locally concentrated when the sealing film 18 is peeled off from the edge of the packaging container 10. Since the bulging portion 52 is curved in a circular arc shape, a part where tensile force is locally concentrated appears even when the sealing film 18 is pulled from various directions. Hence, it is possible to peel off the sealing film 18 more easily than in a case of not providing the bulging portion 52.

The packaging container 10 of the present embodiment is configured as described above, and its operations will be described below.

The packaging container 10 of the present embodiment is provided to the user in a state where the drug solution administration device 12 is accommodated in the recess 14 of the container body 16, and the recess 14 and the drug solution administration device 12 are sealed by the sealing film 18.

The user opens the packaging container 10 immediately before use and takes out the drug solution administration device 12 from the container body 16. When opening the packaging container 10, first, an operation for peeling off the easy-to-peel portion 48 of the seal portion 44 of the sealing film 18 from the sticking portion 32 of the container body 16 is performed.

In the prior art, the entire area of the sealing film 18 is bonded with a uniform peeling strength, and hence the entire sealing film 18 can be peeled off at once. In this case, the entire area of the protection sheet 28 of the drug solution administration device 12 accommodated in the packaging container 10 is exposed. In this case, the peeling start portion 30 of the protection sheet 28 is exposed as a portion easy to pinch when pulling out the drug solution administration device 12. Therefore, there is a risk that the user pulls the peeling start portion 30 in order to pull out the drug solution administration device 12 from the container body 16. Since the protection sheet 28 can be peeled off from the housing attachment material 24 with a weak peeling strength, there is a risk that the protection sheet 28 is peeled off before the drug solution administration device 12 is taken out of the container body 16, and the protection function of the adhesion surface 24b is impaired.

On the other hand, according to the packaging container 10 of the present embodiment, the seal portion 44, by which the sealing film 18 is bonded to the sticking portion 32 of the container body 16, is provided with the difficult-to-peel portion 46 and the easy-to-peel portion 48. In the difficult-to-peel portion 46, the sealing film 18 is bonded to the sticking portion 32 throughout the entire area in the width direction of the sticking portion 32, and hence the difficult-to-peel portion 46 is bonded with a relatively strong peeling strength, and has no pinch margin of the sealing film 18 for starting peeling. Therefore, the user pinches the end of the sealing film 18 from the easy-to-peel portion 48 to start peeling of the sealing film 18.

The separation portion 33 is provided at an end of the container body 16 on the side separated from the difficult-to-peel portion 46. The separation portion 33 is separated from the sealing film 18 and coming off, and hence the user can easily grasp the sealing film 18. Therefore, the packaging container 10 can prompt the user to start peeling the sealing film 18 from a part away from the difficult-to-peel portion 46.

When the user pinches the end of the sealing film 18 and peels it off from the container body 16, the easy-to-peel portion 48 of the sealing film 18 peels along the linear patterns 48a and 48b. The peeling of the sealing film 18 stops when the peeling proceeds to the difficult-to-peel portion 46. In the difficult-to-peel portion 46, since the sealing film 18 and the sticking portion 32 are bonded with a strong peeling strength, the peeling of the difficult-to-peel portion 46 does not proceed any more in normal operation. Therefore, the peeling of the sealing film 18 ends in a state where the sealing film 18 covers the peeling start portion 30 of the protection sheet 28 of the drug solution administration device 12.

This can protect the peeling start portion 30, which is a protection target portion of the drug solution administration device 12. Note that even in a state where the sealing film 18 is not completely peeled off, the user can grasp the housing 20 of the drug solution administration device 12 through the grasping recess 42, and hence the user can remove the drug solution administration device 12 from the recess 14 of the container body 16.

The packaging container 10 of the present embodiment achieves the following effects.

The present embodiment relates to the packaging container 10 for accommodating the drug solution administration device 12 (medical device/stored object) having the peeling start portion 30 (protection target portion) to be avoided from contact when taken out and a graspable portion that can be grasped. The packaging container 10 has: the container body 16 having the recess 14 formed in a shape corresponding to the drug solution administration device 12 and accommodating the drug solution administration device 12, and the sticking portion 32 formed in a plane shape in a part surrounding the periphery of the recess 14; and the sealing film 18 peelably stuck to the sticking portion 32 and sealing the recess 14, in which the sealing film 18 is bonded to the sticking portion 32 via the seal portion 44 extending along the periphery of the recess 14, and the seal portion 44 has the difficult-to-peel portion 46 formed in the part surrounding the peeling start portion 30 (protection target portion) of the drug solution administration device 12 (medical device/stored object) accommodated in the recess 14, and bonded with a strength relatively strong, and the easy-to-peel portion 48 formed in the part surrounding the graspable portion of the drug solution administration device 12 accommodated in the recess 14, and bonded with a strength relatively weaker than that with the difficult-to-peel portion 46.

According to the packaging container 10 described above, when the sealing film 18 is peeled to open the packaging container 10, the peeling of the sealing film 18 is stopped by the difficult-to-peel portion 46. Therefore, it is possible to maintain a state in which the sealing film 18 covers and protects the peeling start portion 30 of the drug solution administration device 12. This can prevent the user from touching the peeling start portion 30 as a protection target portion when the user takes out the drug solution administration device 12 from the container body 16.

In the packaging container 10 described above, the easy-to-peel portion 48 is formed as the thin linear patterns 48a and 48b, and the difficult-to-peel portion 46 may be formed on the entire surface of the sticking portion 32 in a part surrounding the peeling start portion 30 (protection target portion).

This, by separately producing the difficult-to-peel portion 46 and the easy-to-peel portion 48 in accordance with the shape of the seal portion 44, it is possible to simultaneously produce them in one bonding process. This can reduce the number of manufacturing process, and the productivity is excellent.

In the packaging container 10 described above, the easy-to-peel portion 48 has the two parallel linear patterns 48a and 48b as the seal portion 44. According to this configuration, even when the sealing film 18 is about to be peeled off due to an impact or the like, the peeling stops in any one of the linear patterns 48a and 48b, and hence the risk of unintended opening can be reduced.

In the packaging container 10 described above, the linear patterns 48a and 48b have the plurality of straight parts 49a and the bent portion 49b connecting the straight parts 49a, and the bent portion 49b of the linear pattern 48b may be formed with the bulging portion 52 curved and bulging outward. According to this configuration, since the load when the sealing film 18 is pulled in the peeling direction is concentrated on the bulging portion 52, the peeling of the sealing film 18 can be started with a small force.

In the packaging container 10 described above, the bulging portion 52 may be provided in the bent portion 49b farthest apart from the difficult-to-peel portion 46. This can start the peeling of the sealing film 18 from a part away from the difficult-to-peel portion 46, and the peeling operation of the easy-to-peel portion 48 becomes easy.

The packaging container 10 described above may be provided with the separation portion 33 that extends from the sticking portion 32 in the vicinity of the bulging portion 52 and lowers in a direction away from the sealing film 18. According to this configuration, when the sealing film 18 is peeled off, the user can easily grasp the sealing film 18, and can easily peel off the sealing film 18.

The stored object accommodated in the packaging container 10 is the drug solution administration device 12 used by attaching to the body, and the drug solution administration device 12 may have the box-shaped housing 20, a housing attachment material 24 formed to have an outer shape larger than the housing 20 and having the adhesion surface 24b for attaching the housing 20 to the patient's body, and the protection sheet 28 covering the adhesion surface 24b of the housing attachment material 24 and having the peeling start portion 30 separated from the housing attachment material 24 at one end. The drug solution administration device 12 may be accommodated in the recess 14 of the container body 16 of the packaging container 10 in an orientation in which the protection sheet 28 faces the sealing film 18, and the difficult-to-peel portion 46 may be formed in the sticking portion 32 of a part surrounding the peeling start portion 30 of the protection sheet 28.

According to the above configuration, it is possible to protect the peeling start portion 30 of the protection sheet 28 when the drug solution administration device 12 is taken out from the container body 16.

### (Second Embodiment)

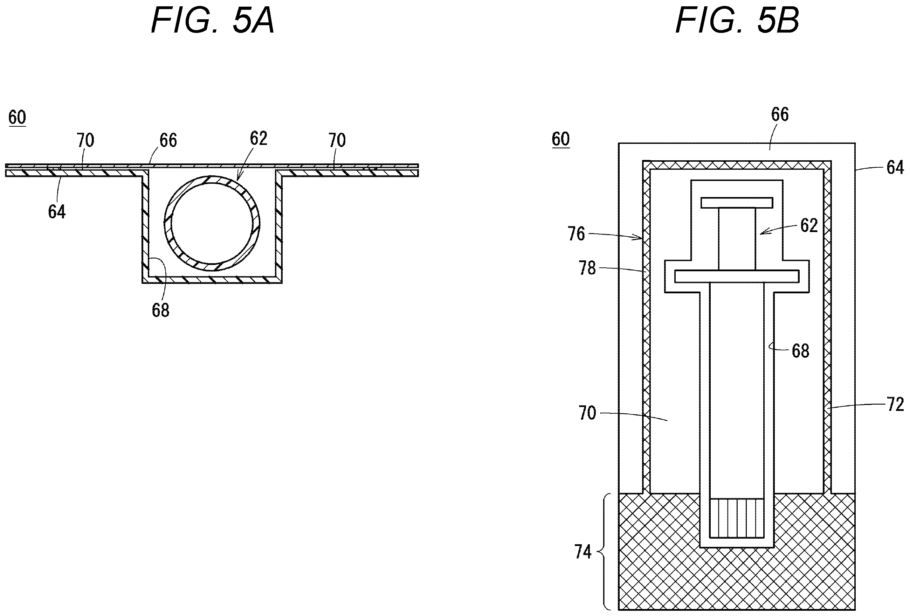

As shown in FIGS. 5A and 5B, the packaging container 60 of the present embodiment is a container that packs a prefilled syringe 62 (medical device). The packaging container 60 includes a container body 64 made of a resin sheet and a sealing film 66 that seals the container body 64. The container body 64 is made of a transparent thick resin sheet made of PET resin or the like, and a recess 68 formed by vacuum molding or the like is formed in the center part thereof. The recess 68 is formed in a shape corresponding to the prefilled syringe 62, and the entire prefilled syringe 62 can be accommodated in the recess 68. A planar sticking portion 70 for bonding the sealing film 66 is formed on the outer peripheral part of the container body 64.

The sealing film 66 and the sticking portion 70 are peelably bonded to each other in a seal portion 72 having a predetermined shape. In the present embodiment, the tip of the prefilled syringe 62 is a protection target portion. Therefore, in the seal portion 72, a difficult-to-peel portion 74 is formed in the sticking portion 70 in a part surrounding the tip of the prefilled syringe 62, and an easy-to-peel portion 76 is formed at another part. The difficult-to-peel portion 74 is formed by bonding the sealing film 66 to the entire surface of the sticking portion 70 of the part surrounding the tip of the prefilled syringe 62. The easy-to-peel portion 76 is formed as a linear pattern 78 with a thin width.

Also in the packaging container 60 of the present embodiment, the user can peel off the sealing film 66 from the easy-to-peel portion 76. The peeling of the sealing film 66 by the user is stopped at the difficult-to-peel portion 74. Thus, the sealing film 66 is kept in a state of covering the tip (protection target portion) of the prefilled syringe 62. It is possible to prevent the user's hand from touching the tip of the prefilled syringe 62 when the user removes the prefilled syringe 62 from the container body 64.

Although preferred embodiments of the present invention have been described above, the present invention is not limited to the embodiments described above, and, needless to say, various modifications can be made

## Claims

1. A packaging container (10) for accommodating a stored object having a protection target portion to be avoided from contact when taken out and a graspable portion that can be grasped, the packaging container (10) comprising:
a container body (16) having a recess (14, 42, 68) formed in a shape corresponding to the stored object and configured to accommodate the stored object and a sticking portion (32) formed in a plane shape in a part surrounding a periphery of the recess (14); and
a sealing film (18) peelably stuck to the sticking portion (32) to seal the recess (14, 42, 68), wherein
the sealing film (18) is bonded to the sticking portion (32) via a seal portion (44) extending along a periphery of the recess (14), and
the seal portion (44) has
a difficult-to-peel portion (46, 74) formed in a part surrounding a part of the recess foreseen for the protection target portion of the stored object to be accommodated in the recess (14, 42, 68), and bonded with a strength relatively strong, and
an easy-to-peel portion (48, 76) formed in a part surrounding a part of the recess foreseen for the graspable portion of the stored object to be accommodated in the recess (14, 42, 68), and bonded with a strength relatively weaker than that with the difficult-to-peel portion (46, 74),
**characterized in that**
the easy-to-peel portion (48, 76) has two parallel linear patterns (48a, 48b) as the seal portion (44).

2. The packaging container (10) according to claim 1, wherein the easy-to-peel portion (47, 76) is formed as a thin linear pattern (48a, 48b), and the difficult-to-peel portion (46, 74) is formed on an entire surface of the sticking portion (32) in a part surrounding the protection target portion.

3. The packaging container (10) according to claim 1 or 2, wherein the linear patterns (48a, 48b) have a plurality of straight parts (49a) and a bent portion (49b) connecting the straight parts (49a), and the bent portion (49b) of the linear pattern (48a, 48b) is formed with a bulging portion curved and bulging outward.

4. The packaging container (10) according to claim 3, wherein the bulging portion (52) is provided in the bent portion (49b) farthest apart from the difficult-to-peel portion (46, 74).

5. The packaging container (10) according to claim 3 or 4, comprising: a separation portion (33) that extends from the sticking portion (32) in a vicinity of the bulging portion (52) and lowers in a direction away from the sealing film (18).

6. The packaging container (10) according to any one of claims 1 to 5, wherein the stored object is a medical device.

7. The packaging container (10) according to any one of claims 1 to 6,
further comprising a stored object,
wherein the stored object is a drug solution administration device (12) used by attaching to a body, and the drug solution administration device (12) has
a box-shaped housing (20),
a housing attachment material (24) formed to have an outer shape larger than the housing and having an adhesion surface (24b) for attaching the housing to a patient's body, and
a protection sheet (28) covering the adhesion surface (24b) of the housing attachment material (24) and having a peeling start portion (30) separated from the housing attachment material (24) at one end,
the drug solution administration device (12) is accommodated in the recess (14, 42, 68) of the container body (16) in an orientation in which the protection sheet (28) faces the sealing film (18), and
the difficult-to-peel portion (46, 74) is formed in the sticking portion (32) of a part surrounding the peeling start portion (30) of the protection sheet (28).

## Patentansprüche

1. Verpackungsbehälter (10) zur Aufnahme eines aufbewahrten Gegenstands, der einen zu schützenden Zielabschnitt aufweist, mit dem bei dem Herausnehmen ein Kontakt zu vermeiden ist, und einen ergreifbaren Bereich, der ergriffen werden kann, wobei der Verpackungsbehälter (10) umfasst:
einen Behälterkörper (16), der eine Aussparung (14, 42, 68), die in einer Form ausgebildet ist, die dem aufbewahrten Gegenstand entspricht, und die so konfiguriert ist, dass sie den aufbewahrten Gegenstand aufnimmt, und einen klebenden Abschnitt (32) aufweist, der in einer ebenen Form in einem Teil ausgebildet ist, der einen Umfang der Aussparung (14) umgibt; und
eine Versiegelungsfolie (18), die abziehbar an dem klebenden Abschnitt (32) angeklebt ist, um die Aussparung zu versiegeln (14, 42, 68), wobei
die Versiegelungsfolie (18) über einen Versiegelungsabschnitt (44), der sich entlang eines Umfangs der Aussparung (14) erstreckt, mit dem klebenden Abschnitt (32) verbunden ist, und
der Versiegelungsabschnitt (44) aufweist
einen schwer abziehbaren Abschnitt (46, 74), der in einem Teil ausgebildet ist, der einen Teil der Aussparung umgibt, der für den zu schützenden Teil des in der Aussparung (14, 42, 68) unterzubringenden aufbewahrten Gegenstands vorgesehen ist, und mit einer relativ starken Festigkeit verbunden ist, und
einen leicht abziehbaren Abschnitt (48, 76), der in einem Teil ausgebildet ist, der einen Teil der Aussparung umgibt, der für den ergreifbaren Abschnitt des in der Aussparung (14, 42, 68) unterzubringenden aufbewahrten Gegenstands vorgesehen ist, und mit einer Festigkeit verbunden ist, die relativ schwächer ist als diejenige des schwer ablösbaren Abschnitts (46, 74),
**dadurch gekennzeichnet, dass**
der leicht abziehbare Abschnitt (48, 76) zwei parallele lineare Muster (48a, 48b) als den Versiegelungsabschnitt (44) aufweist.

2. Verpackungsbehälter (10) nach Anspruch 1, wobei der leicht abziehbare Abschnitt (47, 76) als ein dünnes lineares Muster (48a, 48b) ausgebildet ist und der schwer abziehbare Abschnitt (46, 74) auf einer gesamten Oberfläche des klebenden Abschnitts (32) in einem Teil ausgebildet ist, welcher den zu schützenden Zielabschnitt umgibt.

3. Verpackungsbehälter (10) nach Anspruch 1 oder 2, wobei die linearen Muster (48a, 48b) eine Mehrzahl gerader Teile (49a) und einen gebogenen Abschnitt (49b) aufweisen, der die geraden Teile (49a) verbindet, und der gebogene Abschnitt (49b) des linearen Musters (48a, 48b) mit einem vorgewölbten Abschnitt, der nach außen gewölbt und gekrümmt ist, ausgebildet ist.

4. Verpackungsbehälter (10) nach Anspruch 3, wobei der vorgewölbte Abschnitt (52) in dem von dem schwer abziehbaren Abschnitt (46, 74) am weitesten entfernt liegenden gebogenen Abschnitt (49b) vorgesehen ist.

5. Verpackungsbehälter (10) nach Anspruch 3 oder 4, umfassend: einen Trennabschnitt (33), der sich von dem klebenden Abschnitt (32) in die Nähe des vorgewölbten Abschnitts (52) erstreckt und sich in einer Richtung weg von der Versiegelungsfolie (18) absenkt.

6. Verpackungsbehälter (10) nach einem der Ansprüche 1 bis 5, wobei der aufbewahrte Gegenstand eine medizinische Vorrichtung ist.

7. Verpackungsbehälter (10) nach einem der Ansprüche 1 bis 6, der ferner einen aufbewahrten Gegenstand umfasst,
wobei der aufbewahrte Gegenstand eine Vorrichtung (12) zur Verabreichung einer Arzneimittellösung ist, die durch Anbringen an einem Körper verwendet wird, und die Vorrichtung (12) zur Verabreichung einer Arzneimittellösung aufweist,
ein kastenförmiges Gehäuse (20),
ein Gehäuse-Befestigungsmaterial (24), das so geformt ist, dass es eine Außenform aufweist, die größer ist als das Gehäuse, und eine Klebefläche (24b) zum Befestigen des Gehäuses an dem Körper eines Patienten aufweist, und
eine Schutzfolie (28), welche die Klebefläche (24b) des Gehäuse-Befestigungsmaterials (24) bedeckt und einen Abziehstartabschnitt (30) aufweist, der an einem Ende von dem Gehäuse-Befestigungsmaterial (24) getrennt ist,
wobei die Vorrichtung (12) zur Verabreichung einer Arzneimittellösung in der Aussparung (14, 42, 68) des Behälterkörpers (16) in einer Ausrichtung untergebracht ist, in der die Schutzfolie (28) der Versiegelungsfolie (18) zugewandt ist, und
der schwer abziehbare Abschnitt (46, 74) in dem klebenden Abschnitt (32) eines Teils ausgebildet ist, welcher den Abziehstartabschnitt (30) der Schutzfolie (28) umgibt.

## Revendications

1. Récipient d'emballage (10) pour accueillir un objet stocké ayant une portion cible de protection dont le contact est à éviter lors d'un retrait et une portion saisissable pouvant être saisie, le récipient d'emballage (10) comprenant :
un corps de récipient (16) présentant un évidement (14, 42, 68) formé selon une forme correspondant à l'objet stocké et configuré pour accueillir l'objet stocké et une portion collante (32) formée selon une forme plane dans une partie entourant une périphérie de l'évidement (14) ; et
un film d'étanchéité (18) collé de manière pelable à la portion collante (32) pour étanchéifier l'évidement (14, 42, 68), dans lequel
le film d'étanchéité (18) est lié à la portion collante (32) via une portion joint étanche (44) s'étendant le long d'une périphérie de l'évidement (14), et
la portion joint étanche (44) a
une portion (46, 74) difficile à peler formée dans une partie entourant une partie de l'évidement envisagée pour la portion cible de protection de l'objet stocké à accueillir dans l'évidement (14, 42, 68), et liée avec une résistance relativement forte, et
une portion (48, 76) facile à peler formée dans une partie entourant une partie de l'évidement envisagée pour la portion saisissable de l'objet stocké à accueillir dans l'évidement (14, 42, 68), et liée avec une résistance relativement plus faible que celle avec la portion (46, 74) difficile à peler,
**caractérisé en ce que**
la portion (48, 76) facile à peler présente deux motifs linéaires parallèles (48a, 48b) en tant que portion joint étanche (44).

2. Récipient d'emballage (10) selon la revendication 1, dans lequel la portion (47, 76) facile à peler est formée sous la forme d'un motif linéaire fin (48a, 48b), et la portion (46, 74) difficile à peler est formée sur toute une surface de la portion collante (32) dans une partie entourant la portion cible de protection.

3. Récipient d'emballage (10) selon la revendication 1 ou 2, dans lequel les motifs linéaires (48a, 48b) présentent une pluralité de parties rectilignes (49a) et une portion courbée (49b) reliant les parties rectilignes (49a), et la portion courbée (49b) du motif linéaire (48a, 48b) est pourvue d'une portion bombée courbée et bombée vers l'extérieur.

4. Récipient d'emballage (10) selon la revendication 3, dans lequel la portion bombée (52) est disposée dans la portion courbée (49b) la plus éloignée de la portion (46, 74) difficile à peler.

5. Récipient d'emballage (10) selon la revendication 3 ou 4, comprenant : une portion de séparation (33) qui s'étend à partir de la portion collante (32) à proximité de la portion bombée (52) et s'abaisse dans une direction s'éloignant du film d'étanchéité (18).

6. Récipient d'emballage (10) selon l'une quelconque des revendications 1 à 5, dans lequel l'objet stocké est un dispositif médical.

7. Récipient d'emballage (10) selon l'une quelconque des revendications 1 à 6, comprenant en outre un objet stocké, dans lequel l'objet stocké est un dispositif d'administration de solution médicamenteuse (12) utilisé par fixation à un corps, et le dispositif d'administration de solution médicamenteuse (12) a
un logement (20) en forme de boîte,
un matériau de fixation de logement (24) formé pour avoir une forme externe plus grande que le logement et ayant une surface d'adhérence (24b) pour fixer le logement au corps d'un patient, et
une feuille de protection (28) recouvrant la surface d'adhérence (24b) du matériau de fixation de logement (24) et présentant une portion de début de pelage (30) séparée du matériau de fixation de logement (24) à une extrémité, le dispositif d'administration de solution médicamenteuse (12) est logé dans l'évidement (14, 42, 68) du corps de récipient (16) dans une orientation dans laquelle la feuille de protection (28) fait face au film d'étanchéité (18), et
la portion (46, 74) difficile à peler est formée dans la portion collante (32) d'une partie entourant la portion de début de pelage (30) de la feuille de protection (28).
